# EUROPEAN PATENT SPECIFICATION

(11) **EP 4 245 358 B1**
(45) Date of publication and mention of the grant of the patent: **05.11.2025**
(21) Application number: 23183326.0
(22) Date of filing: 14.05.2020
(51) Int. Cl.: A61N 1/05, A61N 1/36

(54) **SYSTEM FOR CONTROL OF AUTONOMIC FUNCTION**
SYSTEM ZUR STEUERUNG EINER AUTONOMEN FUNKTION
SYSTÈME DE COMMANDE DE FONCTION AUTONOME

(30) Priority: 15.05.2019 EP 19174556
(43) Date of publication of application: 20.09.2023
(62) Divisional of application: 20726109.0
(73) Proprietor: Ecole Polytechnique Fédérale de Lausanne EPFL-TTO, 1015 Lausanne (CH); UTI Limited Partnership, Calgary, AB T2L 1Y8 (CA)
(72) Inventor: Courtine, Grègoire, 1003 Lausanne (CH); Phillips, Aaron, Calgary, Alberta, T2P0X2 (CA); Squair, Jordan, 1003 Lausanne (CH)
(74) Representative: DTS Patent- und Rechtsanwälte PartmbB

(56) References cited:
- WO-A1-2018/148844
- US-A1- 2010 228 310
- US-A1- 2013 289 650
- US-A1- 2014 081 345
- US-A1- 2018 133 480

## Description

The present invention refers to the field of spinal cord neuro-prostheses, in particular for rehabilitation of autonomic functions.

In particular, it refers to a system for stimulation of the spinal cord, more in particular for the rehabilitation of autonomic function, in particular blood pressure, in mammals with spinal cord injury or other disorders (e.g. stroke, multiple sclerosis, autonomic failure, autonomic neuropathy or cancer of the neurological tissue which impair operation of descending sympathetic pathways that normally facilitate control of autonomic functions).

The spinal cord is an integral part of the central nervous system (CNS). Spinal cord injury (SCI) results in motor and sensory deficits but also in autonomic dysfunctions. SCI results in disconnection of some, most, or all descending sympathetic pathways that carry signals responsible for regulating arterial blood pressure, heart rate and/or gut and bladder function.

Autonomic dysfunction following SCI is a potentially life-threatening condition that leads to blood pressure instability and ensuing chronic dysfunction of the heart and vasculature. Most SCI patients experience multiple drastic, blood pressure fluctuations daily, and rank this as a top healthcare priority.

Blood pressure is the pressure of circulating blood on the walls of blood vessels. Without further specification, 'blood pressure' often may refer to the pressure in arteries of the systemic circulation. Blood pressure may usually be expressed in terms of the systolic pressure, i.e. the maximum pressure during one heartbeat and/or over diastolic pressure, i.e. the minimum pressure in between two heartbeat and/or over mean arterial blood pressure, i.e. an average blood pressure in an individual during a single cardiac cycle, and may be measured in millimeters of mercury (mmHg, above the surrounding atmospheric pressure).

Blood pressure monitoring may comprise monitoring a parameter value such as a diastolic blood pressure, a systolic blood pressure, a diastolic blood pressure and a systolic blood pressure, a mean arterial pressure, a blended blood pressure value or the like.

Further, perfusion pressure, i.e. spinal cord perfusion pressure, which is defined as the difference between mean arterial blood pressure and cerebrospinal fluid pressure may be of importance. The latter may be monitored using intrathecal catheters placed e.g. near the site of injury or in the lumbar cistern of the injured mammal.

When blood pressure, in particular arterial blood pressure, decreases or increases as a consequence of SCI, the spinal cord neurons responsible for blood pressure control no longer have the capacity to maintain blood pressure at a normal, physiological level. This disconnection of sympathetic control can lead to a situation where blood vessels do not maintain appropriate tone (e.g. the blood vessels can become dilated). Large amounts of blood may pool in subjects' lower part of the body, e.g. legs and gut. Consequently, subjects affected by SCI can suffer from extremely low blood pressure, i.e. hypotension. Individuals with SCI are often unable to regulate their blood pressure. These individuals typically experience very low arterial blood pressure at rest, during exercise and/or when assuming a seated or standing position. This hypotension may lead to dizziness, disorientation, reduction in cognitive functioning, loss of consciousness and a predisposition to strokes and heart attacks. Additionally, dangerous elevations in blood pressure, i.e. hypertension may also result from SCI. Hypertension may lead to heart attacks, strokes, and sub-clinical vascular consequences. As described above, autonomic cardiovascular dysfunctions following SCI are a top health priority. One primary autonomic issue after high-level SCI (i.e., above the 6th thoracic segment) is orthostatic hypotension, which is clinically-defined as a ≥20 mmHg decrease in systolic blood pressure and/or a 10 mmHg decrease in diastolic blood pressure when assuming the upright posture. Another critical autonomic issue after SCI is autonomic dysreflexia, which is associated with potentially life threatening elevations in blood pressure, due to afferent input activating sympathetic circuitry located caudally on the spinal cord to the location of the SCI. Clinically, autonomic dysreflexia is defined as elevations in systolic blood pressure of 20 mmHg or more (WO2018148844A1).

WO2018148844A1 discloses a device and algorithm for controlling an autonomic function in an individual. In particular, a controller device is disclosed that utilizes physiological measurements (such as blood pressure) to regulate spinal cord electrical stimulation to stabilize blood pressure. A control interface and algorithm for controlling an autonomic function in a subject. In particular, an algorithm that utilizes physiological measurements is disclosed (such as blood pressure) to regulate spinal cord electrical stimulation to stabilize blood pressure. The neuronal structures involved may be located within the T1 to S5 segments of the spinal cord, for example. Stimulation may be configured to control a particular function by selecting electrodes and/or the nature of the stimulation.

US2007156200A1 discloses an apparatus and a method for controlling blood pressure by stimulating the cardiac afferent sympathetic nerves. The invention may be implemented in a medical device having a pressure sensor for sensing blood pressure, an electrode for providing electrical signals to the cardiac afferent sympathetic nerves, and a controller for providing signals to the electrode as a function of blood pressure signals received from the pressure sensor.

US2011082515A1 discloses a neurostimulation device including an external neurostimulator worn by a patient using a bracing element that braces a portion of the patient's body. The external neurostimulator delivers neurostimulation to modulate a cardiovascular function of the patient. Preferably, the external stimulator delivers the neurostimulation transcutaneously to a stimulation target in the patient's body using surface stimulation electrodes placed on the body approximately over the stimulation target.

US2011/0202107A1 relates to an electric stimulation apparatus for treating hypotension of patients suffering from SCI and a method for treating hypotension. The electric stimulation apparatus comprises: a blood pressure measuring means for continuously measuring a blood pressure of a subject; an electric current application means for intermittently applying an electric current to skin of the subject; and a control means for controlling the electric current application means so as to maintain the blood pressure at a predetermined target blood pressure value by activating the electric current application means when the subject blood pressure is equal to or less than the target blood pressure value.

US2013/0289650A1 relates to neuromodulation for controlling hypertension and other cardio-renal disorders of a patient suffering from SCI. A neuromodulation device is delivered to a patient's body for applying electric activation to decrease renal sympathetic hyperactivity of the patient based on monitored blood pressure of the patient, substantially without thermal energization of the patient's body by applying the electric activation. The electric activation may also depend on monitored blood volume of the patient. A feedback control module may be used to provide feedback control information for adjusting the electric activation based on the monitored blood pressure and volume of the patient.

US3650277A discloses a system for reducing and controlling the blood pressure of a hypertensive patient by providing electrical pulse stimulation of the carotid-sinus nerves controlled by the arterial blood pressure of the patient in such a manner that the number of stimulation pulses within each heart cycle is determined by the arterial means blood pressure whereas the distribution of stimulation pulses over the heart cycle is a function of the arterial pulse wave shape with the pulse frequency being greater during the first portion of the heart cycle.

US6058331 discloses techniques for therapeutically treating peripheral vascular disease. A sensor is implemented for sensing the extent of blood flow in a patient's limb or ischemic pain and generating a corresponding sensor signal. The signal is processed to determine the level of spinal cord stimulation or peripheral nerve stimulation to be applied. This information is provided to a signal generator which thereby provides electrical stimulation energy to one or more stimulation leads. Stimulation of the spinal cord, peripheral nerve or neural tissue ganglia thereby improves blood flow, helps restore tissue health and reduces the extent of ischemic pain in the limbs of a peripheral vascular disease patient or organs of other patients. The present invention thereby allows the stimulation to be adjusted automatically to account for changing conditions of the patient throughout the day.

US2007027495A1 relates to an implantable bladder sensor attachable to an exterior surface of a urinary bladder to sense bladder condition or activity for urinary incontinence, or an inability to control urinary function. The sensor includes a strain gauge that detects mechanical deformation of the bladder. Mechanical deformation may be indicative of a gradual filling of the bladder, or an instantaneous contraction indicating an imminent urine voiding event. Wireless telemetry circuitry within the sensor transmits information to implanted electrical stimulator that delivers electrical stimulation for alleviating urinary incontinence, or to an external programmer that controls the implanted stimulator.

There is a need for a new therapy to control and/or manage autonomic dysfunction of subjects after SCI. For the development of such new therapies it is of key importance to understand the sympathetic nervous system connectome after SCI.

It is therefore an object of the present invention to provide a solution for a system and method that can better manage autonomic dysfunction after SCI. The key limitation to the development of such therapeutics, i.e. that the sympathetic nervous system connectome after SCI is not well understood, has to be override. In particular, a rationally targeted stimulation electrode has to be designed in a way that the electrode specifically targets sympathetic nervous system structures responsible for blood pressure control and a stimulation paradigm has to be provided that enables precise and biomimetic control over blood pressure after SCI.

This object is solved by the system according to claim 1.

In particular, a system for neuromodulation and/or neurostimulation, for the treatment of a mammal, at least comprises:
at least one control unit configured and arranged to provide stimulation data;
at least one stimulation unit configured and arranged to provide a stimulation pulse;
at least one real-time monitoring unit;
at least one signal-processing unit;
wherein the system is configured and arranged for control of blood pressure, wherein the stimulation unit is constructed to comprise a lead, and
wherein the lead is capable and configured to provide stimulation to the spinal cord at level T9-L1.

The invention is based on the basic idea that a stimulation system has to be provided which specifically targets and modulates sympathetic pre- and post- ganglionic neurons responsible for blood pressure control in a way that enables precise control over blood pressure after SCI. In particular, a unique electrode design is provided, in particular being configured and arranged for being implanted at a specific location of the spinal cord to target the posterior roots, combined with novel stimulation paradigms and closed-loop controllers that specifically target and modulate sympathetic pre- and post- ganglionic neurons responsible for blood pressure control through the modulation of the posterior roots, in a way that enables precise control over blood pressure after SCI.

The real-time monitoring unit may comprise at least one sensor unit. In particular, the real-time monitoring unit may comprise at least one sensor unit configured and arranged to measure and/or monitor blood pressure and/or perfusion pressure of a mammal. The sensor unit may generally measure and/or monitor systolic and/or diastolic and/or mean arterial pressure and/or cerebrospinal fluid pressure (and/or also spinal cord perfusion pressure) of the mammal. It is also possible that the sensor unit also reports pulse rate. The at least one sensor unit may be invasive or non-invasive. In other words, the at least one sensor unit may be at least partially implantable and/or implanted. Alternatively, the at least one sensor unit may be not implanted and/or not implantable.

In general, the sensor unit may comprise at least one sensor and/or at least one sensor base station. The at least one sensor may be a digital or analog sensor system.

It is generally possible, that at least two sensors form a sensor network. A sensor network may generally comprise both at least one at least partially implanted and/or implantable sensor and at least one non-implantable and/or non-implanted sensor.

An implanted and/or implantable sensor and/or a non-implantable and/or non-implanted sensor may be, but is not limited to, an upper arm blood pressure monitor system or a wrist blood pressure monitor system or a finger blood pressure monitor system. The sensor may measure and/or monitor a blood pressure signal indicative for a blood pressure measurement. In general, the at least one sensor may provide continuous monitoring of blood pressure and/or sporadic monitoring of blood pressure and/or measuring or monitoring blood pressure in preset time intervals.

In particular, the blood pressure sensor may be an invasive arterial line. In particular, the invasive arterial line may monitor blood pressure directly and in real-time.

The signal-processing device may compare a blood pressure signal indicative for the blood pressure and/or a signal and/or value and/or a marker that correlates with spinal cord oxygenation of a mammal to a predetermined blood pressure and/or oxygenation target value and/or predetermined blood pressure and/or oxygenation target range stored in the control unit. If the comparison indicates that the blood pressure measurement deviates to a predetermined degree from the predetermined blood pressure target value or is not within a predetermined target blood pressure range, the system, in particular the control unit adapts stimulation parameters in order to restore the blood pressure in a way that the blood pressure is within the predetermined target blood pressure range and/or closed to the predetermined target blood pressure value. This means that if the measured blood pressure value is below the predetermined target blood pressure value and/or range the method increases the level of a stimulation control signal until the blood pressure measurement is in the target blood pressure range and/or matches the predetermined blood pressure value. If the comparison indicates that the blood pressure measurement is above the predetermined target blood pressure range the method decreases the level of the stimulation until the blood pressure measurement matches the predetermined target blood pressure value and/or blood pressure range. In other words, the system may be a closed-loop system.

Further, there may be at least one other sensor. In particular, the system may comprise at least one sensor configured and arranged to measure and/or monitor blood pressure and/or perfusion pressure of a patient, in particular spinal cord perfusion pressure. The sensor may generally measure and/or monitor systolic and/or diastolic and/or mean arterial pressure and/or cerebrospinal fluid pressure (and/or also spinal cord perfusion pressure) of the patient. It may be also possible that the sensor also may report pulse rate. Further, the sensor may be configured and arranged to measure and/or monitor a signal and/or a value and/or a marker that correlates with spinal cord oxygenation. The at least one sensor unit may be invasive or non-invasive. In other words, the at least one sensor may be at least partially implantable and/or implanted. Alternatively, the at least one sensor may be not implanted and/or not implantable.

In principle, the target blood pressure value and/or target blood pressure range may be predetermined by a patient and/or a medical professional (e.g. a therapist, a nurse, a physiotherapist, a physician, a pharmacist, a physician-aid or any other trained operator). It may be also possible, that the target-blood pressure and/or oxygenation value and/or target blood pressure and/or oxygenation range may be changed and/or reset at any time point. The system may be configured and arranged that different target blood pressure values and/or target blood pressure ranges exist, adapted to e.g. a circadian rhythm of a patient.

Alternatively, the system may be an open-loop system.

In general, stimulation may be delivered to the dorsal aspect of the spinal cord of a mammal. The stimulation may affect dorsal roots, dorsal afferent fibres and/or intraspinal structures that are connected directly or indirectly to sympathetic preganglionic neurons that affect the function being controlled.

The stimulation may be provided by electrical stimulation. In particular, the stimulation may be provided by a lead comprising one or multiple electrodes. The lead may be implanted. Alternatively, and/or additionally, transcutaneous stimulation by a lead is also generally possible.

Stimulation may be delivered epidurally (by epidural electrical stimulation, EES) and/or subdurally.

The stimulation unit may be constructed to comprise a lead.

Because of the complexity of the spinal cord, delivering epidural and/or subdural stimulation on the multi-electrode array (lead) implanted is quite challenging.

The lead may be designed and/or constructed for being capable to target cardiovascular and/or blood pressure hotspots of the spinal segments. The cardiovascular hotspots and/or blood pressure hotspots may be identified by functional mapping.

Functional mapping may be understood as follows: To identify the optimal location on the spinal cord to elicit blood pressure responses, a functional mapping procedure may be performed, each segment of the spinal cord from T5 to L2 may be stimulated by targeting the posterior roots projecting to these segments, and blood pressure responses to stimulation may be recorded. The mapping may be performed in an animal model, e.g. a rat model of spinal cord injury. In doing so, it is possible to identify those spinal segments optimal for blood pressure control. The results obtained by the animal model may later be transferred to human beings. The mapping may also be conducted in human beings during surgical intervention using percutaneous leads.

Further, the density of sympathetic pre-ganglionic neurons in the spinal cord projecting to key splanchnic ganglia in the abdomen, which are responsible for blood pressure control may be determined to identify optimal stimulation sites.

Based on the results of the mapping and/or the determination of the density of the sympathetic pre-ganglionic neurons in the spinal cord, the dimension(s), configuration and/or the shape of the lead may be designed and/or constructed.

In particular, the lead may be designed and/or constructed to specifically target the posterior roots of the T9-L1 spinal segments.

In particular, the lead may be capable and configured to provide stimulation to the spinal cord at level T9-T12. In other words, the lead may be designed and constructed to specifically target the posterior roots of the T9-T12 spinal segment.

As described above, the lead may be implanted. The lead may be capable and configured to be positioned subdurally and/or epidurally at least partially at and/or between the level of vertebrae T9-L1, in particular at least partially under vertebrae T9-L1. For epidural stimulation, the lead may be positioned in the epidural space above the spinal cord. For subdural stimulation, the lead may be positioned in the subdural space. In other words, the shape and/or the dimension(s) and/or the configuration of the lead may be constructed in a way that the lead may be positioned subdurally and/or epidurally at least partially at and/or between the level of vertebrae T9-L1, in particular at least partially under vertebrae T9-L1. In particular, specific markers may be placed on the lead to align the lead to these specific vertebral locations. It is generally possible to use diagnostic tools, such as a computer tomography (CT), X-Ray and/or magnetitic resonance imaging (MRI) scan, to align the location of the lead to the specific vertebral location.

The stimulation unit may comprise at least one of a neurostimulator, a neuromodulator and a pulse generator, in particular an implantable pulse generator (IPG). The neurostimulator may be connected to the lead.

The stimulation data may comprise at least frequency, amplitude and pulse width. The frequency may be 10Hz-10kHz, the amplitude may be 0-1A or 0-15V, and the pulse width may be 1-500µs.

Further, the stimulation unit may be configured and arranged to provide at least one burst train stimulation pulse.

It is generally possible that the stimulation unit may provide two or more bursts.

Stimulation pulse trains called burst train stimulation pules may be preferred to increase the specificity and comfort of stimulation. Burst train stimulation may comprise a series of several pulses delivered.

In particular, the stimulation unit may be configured and arranged to provide at least one burst of several pulses, preferably of 2 to 5 pulses.

In particular, burst train stimulation may comprise a series of 3 to 5 (or even more) pulses delivered at e.g. 200Hz to 700Hz, repeated at a frequency of e.g. 10-120Hz.

Further, the control unit may comprise an oscillation control module, wherein the oscillation control module may be configured and arranged to provide an input of 0.01Hz -0.2Hz low frequency oscillation in the amplitude and/or frequency.

In particular, the oscillation control module may be configured and arranged to provide an input of 0.1Hz low frequency oscillation in the amplitude and/or frequency.

In particular, the oscillation control module may mimic the natural state of the intact sympathetic nervous system. Specifically, low frequency oscillation overlays may be optimized, which originate in supraspinal structures responsible for blood pressure and/or oxygenation control (i.e. the rostral ventrolateral medulla), by input of the above described low frequency oscillation in the amplitude or frequency control of the stimulation.

Further, the control unit may comprise a time control module, wherein the time control module may be configured and arranged to provide a time delay.

In particular, the time delay provided by the time control module may be a time delay of 1-50ms.

In particular, the time delay provided by the time control module may be a time delay of 1-4ms, in particular a time delay of 2ms.

Action potentials originate in the rostral ventrolateral medulla and travel with a certain time delay between key thoracic segments. In rats, key thoracic segments may be T11-T12 and T12-T13. In humans, key thoracic segments may be slightly different, i.e. T9-T10, T10-T11 or T11-T12 or T12-L1.

In particular, the time delay may depend on segment length.

In particular, the time delay may be longer for large mammals compared to small mammals. In particular, the time delay may be longer in humans compared to rats or mice.

The time delay may be 1-50ms,
However, other scales for a time delay are generally possible.

For rats, the time delay may be 1-4ms, preferably 2ms.

In other words, the time control module may reproduce the natural supraspinal sympathetic drive, with the aim to deliver biomimetic stimulation patterns. When coupled to standard stimulation parameters, this may enable achieving the best control over blood pressure after spinal cord injury.

Alternatively, and/or additionally, the stimulation unit may provide stimulation by an optical signal, a magnetic signal, optogenetic manipulation, chemogenetic manipulation, stimulation by a chemical or pharmacological agent, a thermal signal, or the like.

The non-claimed use of a system for neuromodulation and/or neurostimulation according to the system of any of claims 1 to 14 for treating a mammal is disclosed.

In particular, the non-claimed method can be a method for neuromodulation, especially a method for neuromodulation and/or neurostimulation to the nervous system of a mammal, at least comprising the steps of
- positioning a lead 20;
- providing neuromodulation and/or neurostimulation to the spinal cord via said lead 20;
- monitoring blood pressure;
- comparing a measured blood pressure value to a pre-set blood pressure target range;
- if the comparison indicates that the measured blood pressure deviates from the target blood pressure range modulating neurostimulation until the blood pressure of the mammal is in the target blood pressure range;
wherein the method is configured and arranged to provide neuromodulation and/or neurostimulation to the spinal cord at spinal level T9-L1.

The system and method may be used in a close-loop fashion, taking into account parameters, in particular parameters indicating blood pressure and/or oxygenation (especially spinal cord oxygenation) and/or perfusion pressure of a patient, in particular spinal cord perfusion pressure. Further, instead of monitoring blood pressure, additionally or alternatively oxygenation can be monitored and compared with target values.

It is generally possible that the system and method may also be used in an open-loop fashion.

Alternatively, and/or additionally, the system may be used for restoring any other type of autonomic dysfunction, such as digestion, bladder and gut control and the like.

Further details and advantages of the present invention shall now be disclosed in connection with the drawings.

It is shown in
- Fig. 1: a schematical overview of an embodiment of the system for neuromodulation and/or neurostimulation according to the present invention, with which the method according to the present invention can be performed;
- Fig. 2: an example of a patient equipped with the system shown in Fig. 1;
- Fig. 3a: an example of a lead of the system shown Fig. 1, and an embodiment for the implantation side of said lead;
- Fig. 3b: a further example to the implantation side to Fig. 3a (rat model);
- Fig. 3c: a further example to the implantation side to Fig. 3a (non-human primate/monkey model);
- Fig. 3d: a further example to the implantation side to Fig. 3a (human patient);
- Fig. 4a: an example for functional mapping results - heatmaps demonstrating preferential increases in SBP, DBP, and MAP at T12, going from acute to chronic injuries;
- Fig. 4b: an example for functional mapping results - quantification of responses at the intermediate stage, showing preference for T11- T13 across all measures;
- Fig. 4c: an example for functional mapping results - quantification of the responses at T12 over time, showing an increase with time post-injury;
- Fig. 4d: functional mapping in a non-human primate;
- Fig. 5: activation of natural frequency aspects within the systolic blood pressure signal during an orthostatic stimulus in uninjured and spinal cord injury rats;
- Fig. 6: activation of natural frequency aspects within the systolic blood pressure signal using biologically relevant stimulation;
- Fig. 7a: a schematical overview of the activation of sympathetic neurons according to the present invention; and
- Fig. 7b: a schematical overview of mechanisms, by which EES stabilizes hemodynamics.

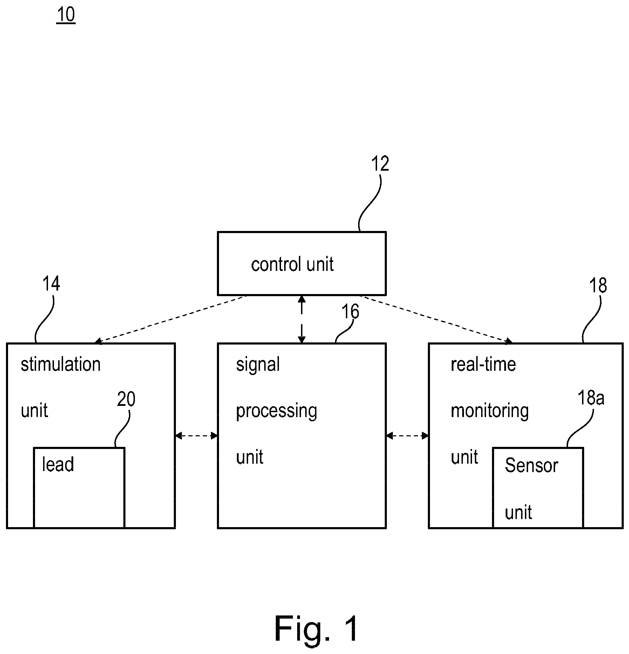

**Fig. 1** shows a schematical overview of an embodiment of the system 10 for neuromodulation and/or neurostimulation, for the treatment of a mammal, according to the present invention, with which the method according to the present invention can be performed.

The system 10 comprises a control unit 12.

The control unit 12 is configured and arranged to provide stimulation data.

Further, the system 10 comprises a stimulation unit 14.

The stimulation unit 14 is configured and arranged to provide a stimulation pulse.

The stimulation unit 14 is constructed to comprise a lead 20.

In this embodiment the lead is capable and configured to provide stimulation to the spinal cord at level T9-L1.

The system 10 also comprises a signal processing unit 16.

The system 10 also comprises a real-time monitoring unit 18.

In this embodiment, the real-time monitoring unit 18 is configured and arranged to monitor blood pressure.

In this embodiment, the real-time monitoring unit 18 comprises a sensor unit 18a.

The sensor unit 18a is configured and arranged to measure and/or monitor blood pressure of a patient P.

In an alternative embodiment, the system 10 may comprise more than one control unit 12 and/or more than one stimulation unit 14 and/or more than one signal processing unit 16 and/or more than one real-time monitoring unit 18.

In this embodiment, the control unit 12 is connected to the stimulation unit 14, the signal processing unit 16 and the real-time monitoring unit 18.

In this embodiment, the connection between the control unit 12 and the stimulation unit 14, the control unit 12 and the processing unit 16 and the control unit 12 and the real-time monitoring unit 18 is a direct and bidirectional connection.

In this embodiment, the connection between the control unit 12 and the stimulation unit 14, the control unit 12 and the processing unit 16 and the control unit 12 and the real-time monitoring unit 18 is established by a wireless link WL.

However, alternatively, also a cable bound and/or unidirectional and/or indirect connection between the control unit 12 and the stimulation unit 14, the control unit 12 and the processing unit 16 and the control unit 12 and the real-time monitoring unit 18 could be generally possible.

In this embodiment, the stimulation unit 14 is connected to the signal processing unit 16.

The connection between the stimulation unit 14 and the signal processing unit 16 is a direct and bidirectional connection.

The connection between the stimulation unit 14 and the signal processing unit 16 is established by a wireless link WL.

However, alternatively, also a cable bound and/or unidirectional and/or indirect connection between the stimulation unit 14 and the signal processing unit 16 could be generally possible.

In this embodiment, the signal processing unit 16 is connected to the real-time monitoring unit 18.

The connection between the signal processing unit 16 and the real-time monitoring unit 18 is a direct and bidirectional connection.

In this embodiment, the connection between the signal processing unit 16 and the real-time monitoring unit 18 are established by a wireless link WL.

However, alternatively, also a cable bound and/or unidirectional and/or indirect connection between the signal processing unit 16 and the real-time monitoring unit 18 unit could be generally possible.

The real-time monitoring unit 18, in particular the sensor unit 18a of the real-time monitoring unit 18 measures blood pressure of the patient P.

Not shown in this embodiment is that the sensor unit 18a could generally measure and/or monitor systolic and/or diastolic and/or mean arterial pressure.

Not shown in this embodiment is that the sensor unit 18a could also reports pulse rate.

Not shown in this embodiment is that the at least one sensor unit 18a may be an invasive or non-invasive sensor unit 18a.

Not shown in this embodiment is that the sensor unit 18a could be at least partially implantable and/or implanted.

Alternatively, the at least one sensor unit 18a could be not implantable and/or not implanted.

The measured blood pressure is communicated from the real-time monitoring unit 18 to the signal processing unit 16.

In this embodiment, the measured blood pressure is communicated from the real-time monitoring unit 18 to the signal processing unit 16 in real-time.

In an alternative embodiment, the measured blood pressure could communicate from the real-time monitoring unit 18 to the signal processing unit 16 closed to real-time or with time delay.

The signal processing unit 16 compares the measured blood pressure value to a predetermined blood pressure value and/or to a predetermined blood pressure target range.

If the comparison indicates that the measured blood pressure deviates from the predetermined target blood pressure range, the control unit 12 could adapt stimulation data.

The stimulation unit 14 provides stimulation via the lead 20 according to the stimulation data provided by the control unit 12.

Not shown in this embodiment is that additionally and/or alternatively, a sensor unit 18a could measure and/or monitor perfusion pressure, in particular spinal cord perfusion pressure.

Not shown in this embodiment is that the measured spinal cord perfusion pressure could be communicated from the real-time monitoring unit 18 to the signal processing unit 16.

Not shown in this embodiment is that the measured spinal cord perfusion pressure could be communicated from the real-time monitoring unit 18 to the signal processing unit 16 in real-time.

Not shown in this embodiment is that the measured spinal cord perfusion pressure could be communicated from the real-time monitoring unit 18 to the signal processing unit 16 closed to real-time or with time delay.

Not shown in this embodiment is that the signal processing unit 16 could compare the measured spinal cord perfusion pressure value to a predetermined spinal cord perfusion pressure target value and/or to a predetermined spinal cord perfusion pressure target range.

Not shown in this embodiment is that if the comparison indicates that the measured spinal cord perfusion pressure deviates from the predetermined spinal cord perfusion pressure target value and/or from the predetermined spinal cord perfusion pressure target range, the control unit 12 could adapt stimulation data.

In this embodiment, the stimulation data comprises at least frequency, amplitude and pulse width.

In this embodiment, the frequency may be 10Hz-10kHz, the amplitude may be 0-1A or 0-15V, and the pulse width may be 1-500µs.

Not shown in this embodiment that the stimulation unit 14 may be configured and arranged to provide at least one burst train stimulation pulse.

Not shown in Fig. 1 is that it could be generally possible that the stimulation unit 14 may provide two or more bursts.

In particular, the stimulation unit 14 could be configured and arranged to provide at least one burst of several pulses, preferably of 2 to 5 pulses.

Not shown in Fig. 1 is that stimulation pulse trains called burst train stimulation could be preferred to increase the specificity and comfort.

Not shown in Fig. 1 is that in particular, burst train stimulation could comprise a series of 3 to 5 (or even more) pulses delivered at e.g. 200Hz to 700Hz, repeated at a frequency of e.g. 10-120Hz.

Not shown in this embodiment is that the lead 20 is capable and configured to provide stimulation to the spinal cord at level T9-T12.

Not shown in this embodiment is that the lead 20 is capable and configured to be positioned subdurally and/or epidurally at least partially at and/or between the level of vertebrae T9-L1, in particular at least partially under vertebrae T9-L1 of the patient P.

Not shown in Fig. 1 is that the control unit 12 could comprise an oscillation control module.

In general, the oscillation control module could provide an input of 0.01Hz-0.2Hz low frequency oscillation in the amplitude.

In particular, the oscillation control module could provide an input of 0.1Hz low frequency oscillation in the amplitude.

Alternatively, and/or additionally, the oscillation control module could provide an input of 0.01Hz-0.2Hz low frequency oscillation in the frequency.

In particular, alternatively and/or additionally, the oscillation control module could provide an input of 0.1Hz low frequency oscillation in the frequency.

Not shown in Fig. 1 is that the control unit 12 could comprise a time control module.

In general, the time control module could provide a time delay.

In general, the time delay could depend on segment length.

In general, the time delay could be longer for large mammals compared to small mammals. In general, the time delay could be longer in humans compared to rats or mice.

In general, the time delay provided by the time control module could be a time delay of 1-50ms. In general, the time delay provided by the time control module could be a time delay of 1-4ms, in particular a time delay of 2ms.

However, every other time delay provided by the time control module could be generally possible.

The non-claimed use of a system 10 or neuromodulation is disclosed.

The use of the system 10 and functionality of the system 10 can be described as follows:
The non-claimed method performed with the system 10 and functionality of the system 10 can be described as follows:
A method for neuromodulation and/or neurostimulation to the nervous system of a mammal, at least comprising the steps of
- positioning a lead 20;
- providing neuromodulation and/or neurostimulation to the spinal cord via said lead 20;
- monitoring blood pressure;
- comparing a measured blood pressure value to a pre-set blood pressure target range;
- if the comparison indicates that the measured blood pressure deviates from the target blood pressure range modulating neurostimulation until the blood pressure of the mammal is in the target blood pressure range;
wherein the method is configured and arranged to provide neuromodulation and/or neurostimulation to the spinal cord at spinal level T9-L1.

In particular, the method could be arranged for positioning the lead subdurally and/or epidurally at least partially at and/or between the level of vertebrae T9-L1, in particular at least partially under vertebrae T9-L1.

In particular, the method may be configured and arranged to provide neuromodulation and/or neurostimulation to the spinal cord at least at spinal level T9-T12.

The method could provide neuromodulation and/or neurostimulation to the spinal cord with a frequency of 10Hz-10kHz, an amplitude of 0-1A or 0-15V, and the pulse width of 1-500µs.

In general, stimulation could be provided to the spinal cord by at least one burst train stimulation pulse.

In general, stimulation could be provided to the spinal cord with at least one burst of several pulses, preferably of 2 to 5 pulses.

In general, stimulation could be provided with a control input of 0.01Hz-0.2Hz low frequency oscillation in the amplitude and/or frequency.

In particular, stimulation could be provided with a control input of 0.1Hz low frequency oscillation in the amplitude and/or frequency.

Further, stimulation could be provided with a control input comprising a time delay.

In general, the time delay could be longer in humans compared to rats or mice.

In general, the time delay could depend on segment length.

In particular, the stimulation could be provided with a control input comprising a time delay of 1-50ms.

In particular, the stimulation could be provided with a control input comprising a time delay of 1-4ms.

In particular, the stimulation could be provided with a control input comprising a time delay of 2ms.

Of note, the present system 10 and method could also be applied for the treatment of a mammal suffering from neurological conditions other than SCI, including but not limited to stroke, multiple sclerosis, autonomic failure, autonomic neuropathy, as well as cancer of the neurological tissue which impair operation of descending sympathetic pathways that normally facilitate control of autonomic functions.

Not shown in Fig. 1 is that the present system and method could also be applied for the treatment of any other autonomic dysfunction than impaired blood pressure control, including but not limited to heart rate, digestive function, bladder control and/or bowel control.

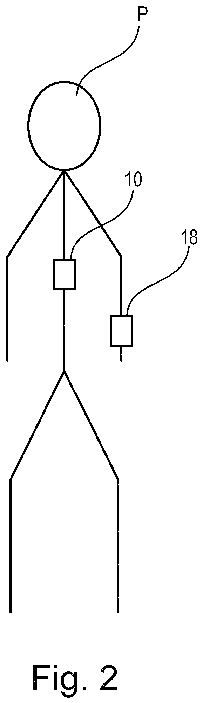

**Fig. 2** shows an example of a patient equipped with the system 10 shown in Fig. 1.

The patient P is equipped with the system 10 as disclosed in Fig. 1.

In this embodiment, the real-time monitoring unit 18 comprises a sensor unit 18a.

In this embodiment, the sensor unit 18a comprises a sensor.

The sensor monitors blood pressure of the patient P.

In this embodiment, the sensor monitors systolic diastolic blood pressure of the patient P.

In this embodiment, the sensor is a non-invasive sensor.

In this embodiment, the sensor is wrist blood pressure monitor system.

In this embodiment, the sensor is a digital blood pressure monitor system.

In an alternative embodiment, the sensor could be an analog blood pressure monitor system.

In this embodiment, the sensor monitors blood pressure continuously in real-time and provides blood pressure data in real-time to the signal processing unit 16.

However, it is generally possible that the sensor measures blood pressure in predefined time-intervals.

In an alternative embodiment, the sensor may be or may comprise other embodiments of blood pressure monitor systems, including but not limited to a cuff, an arterial pressure sensor, an optical biometric sensor, an upper arm blood pressure monitor system, a finger blood pressure monitor system or any other type of non-implanted blood pressure monitor system and any type of implantable and/or implanted blood pressure monitor system.

In an alternative embodiment, the sensor could measure and/or monitor additionally and/or alternatively other parameters indicating perfusion pressure and/or blood pressure, including but not limited to arterial blood pressure.

In an alternative embodiment, the sensor could measure and/or monitor additionally the pulse rate of the patient P.

In an alternative embodiment, the sensor could be or could comprise an arterial line.

In an alternative embodiment, the sensor could be or could comprise an arterial line in the hospital.

In general, the sensor unit 18a could comprise more than one sensor and/or at least one sensor base station.

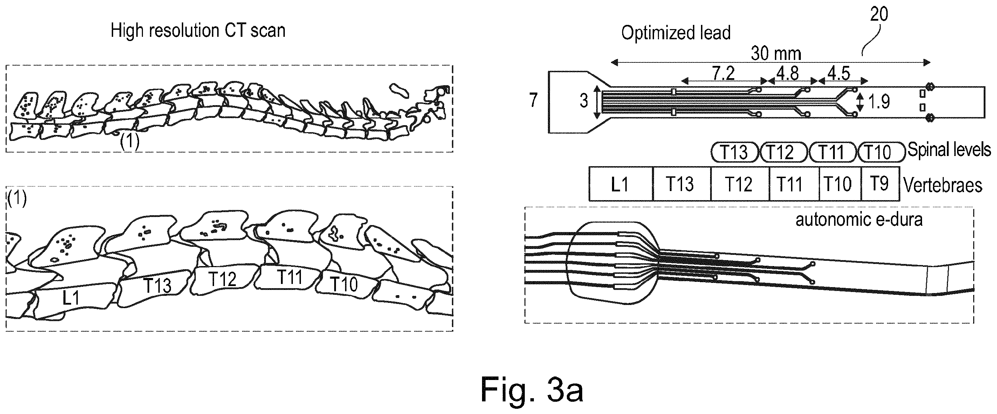

**Fig. 3a** shows an example of a lead 20 of the system 10 shown in Fig. 1, and an embodiment for the implantation side of said lead 20 according to the present invention.

In this embodiment, the dimensions of the lead 20 are designed to perfectly target the posterior roots of the T9-L1 spinal segments.

To identify the optimal location on the spinal cord to elicit blood pressure responses, a functional mapping procedure could be performed, e.g. in an animal model, e.g. in an animal model of SCI.

In other words, to identify the optimal location for providing stimulation to the spinal cord, a functional mapping procedure could be performed, e.g. in an animal model, e.g. in an animal model of SCI.

In other words, to identify the optimal location for positioning a lead 20 for providing stimulation to the spinal cord, a functional mapping procedure could be performed, e.g. in an animal model, e.g. in an animal model of SCI.

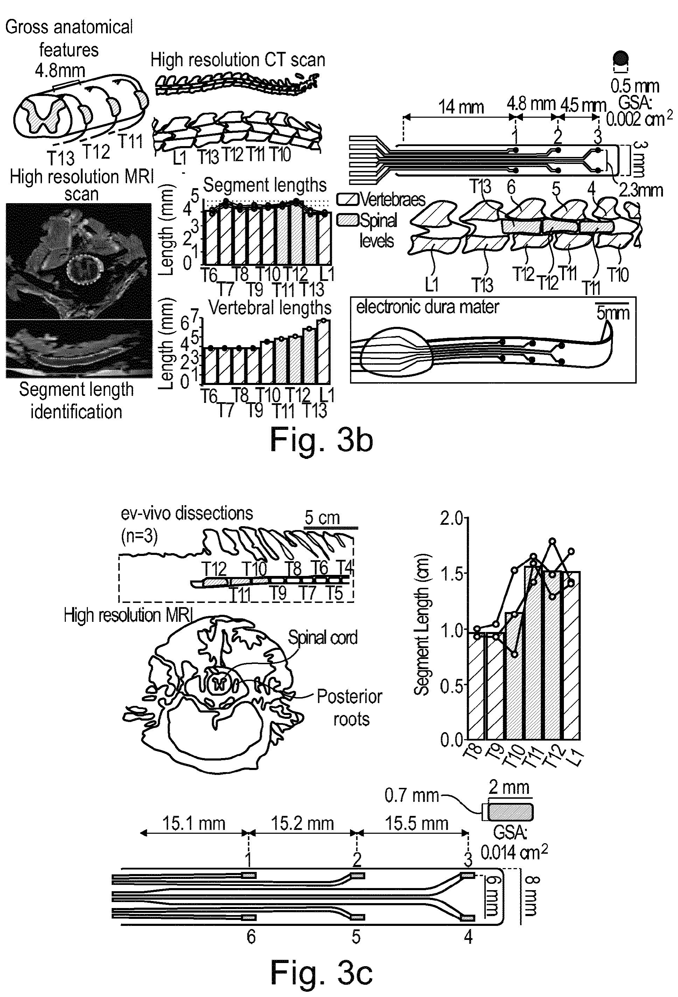

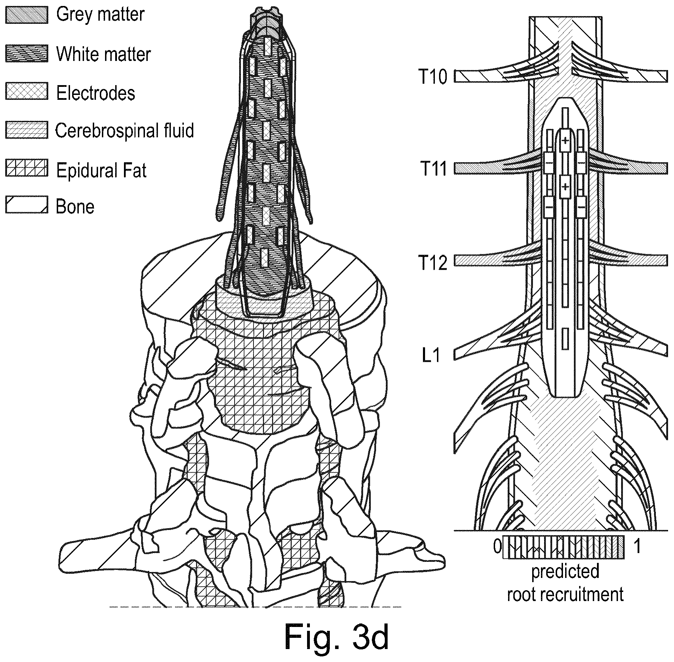

The implantation site is a so-called hotspot and preserved across species, as can be further derived from **Fig. 3b, Fig. 3c** and **Fig. 3d****:**
Fig. 3b shows a further example to the implantation side to Fig. 3a (rat model).
Fig. 3c shows a further example to the implantation side to Fig. 3a (non-human primate/monkey model).
Fig. 3d shows a further example to the implantation side to Fig. 3a (human patient).

In these examples, to identify the optimal location on the spinal cord to elicit blood pressure responses, a functional mapping procedure was performed in a rat model of SCI as described here:
In principle, each segment of the spinal cord from T5 to L2 in a rat model of SCI was stimulated and blood pressure responses to monopolar, 50Hz stimulation were recorded.

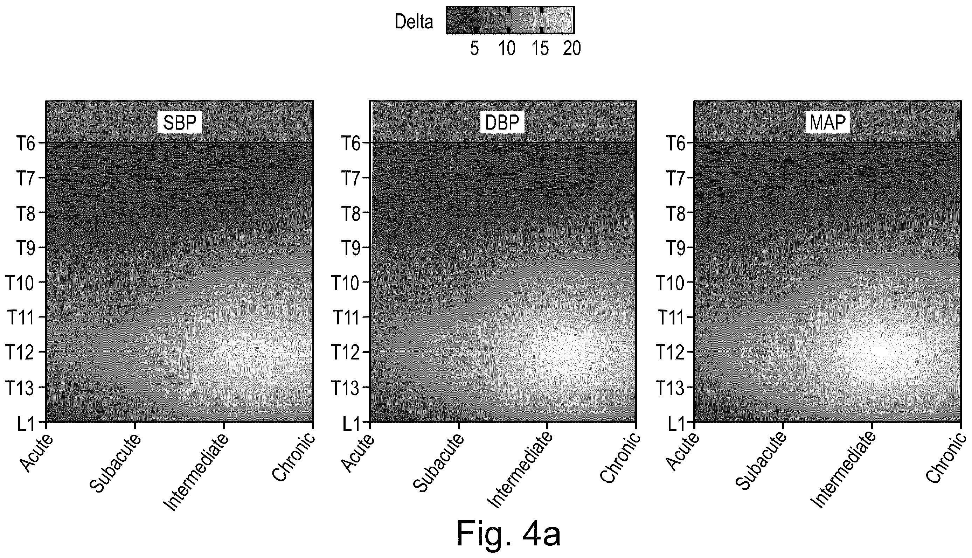

In this example, it has been found that T11-T13 are the optimal segments to stimulate in rodents, with the peak response occurring during stimulation of T12, cf. **Fig. 4a****.**

In particular, systolic blood pressure SBP, diastolic blood pressure DBP or mean arterial blood pressure MAB has been measured.

In particular, this response was consistent across different time-points post injury, including 1 hour (acute), 5 days (subacute), 2 weeks (intermediate), and 1 month (chronic).

Note that a similar approach can be performed in order to obtain a lead 20 designed to perfectly target the posterior roots of the T9-L1 spinal segments of a human.

Note that simulation parameters could vary in a similar approach performed in a human.

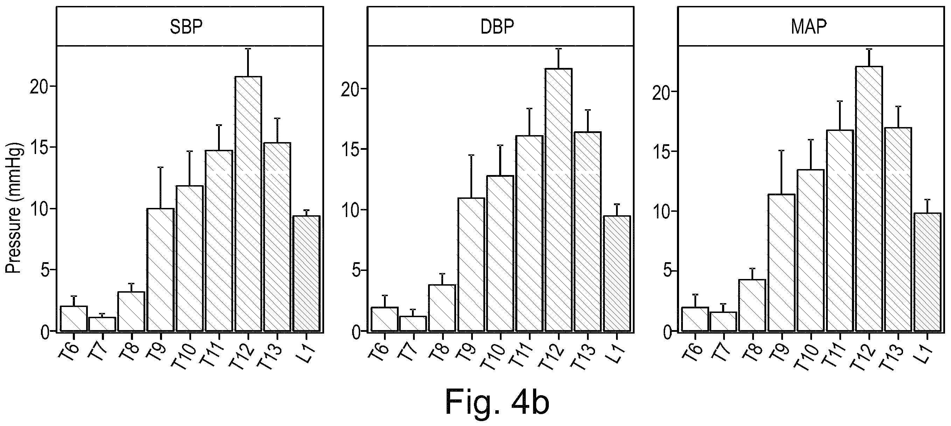

**Fig. 4b** shows bar diagrams showing quantification of responses at the intermediate stage shown in Fig. 4A, showing preference for T11- T13 across all measures.

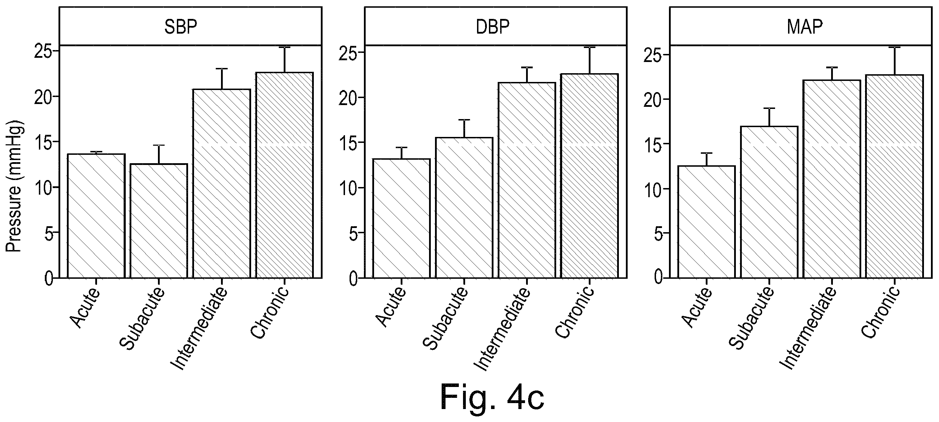

**Fig. 4c** shows bar diagrams showing quantification of the responses at T12 over time post injury, including 1 hour (acute), 5 days (subacute), 2 weeks (intermediate), and 1 month (chronic), showing an increase with time post-injury.

Not shown is that in a next step, the density of sympathetic pre-ganglionic neurons in the spinal cord projecting to key splanchnic ganglia in the abdomen was determined, which is responsible for blood pressure control.

Not shown is that it has also been found that the density of these ganglionic-projecting sympathetic pre-ganglionic neurons peaked in T12, and that there is a strong linear correlation between the density of sympathetic pre-ganglionic neurons and the functional blood pressure response to stimulation.

Not shown is that next, it has been confirmed that this stimulation led to activation of the splanchnic ganglia using two converging lines of evidence.

First, the spinal cord was stimulated at T12 for 30 minutes and classic immunohistochemistry was used to identify active neurons (using the immediate early gene Fos, Fos Proto-Oncogene, AP-1 Transcription Factor Subunit) within the splanchnic ganglia.

Compared to non-stimulated animals, a significant increase in the number of neurons expressing Fos was found, and these neurons were adrenalin-synthesizing (confirmed by the presence of the protein Tyrosine Hydroxylase), confirming their role in blood pressure control.

Using optogenetic techniques, it has been found that inhibiting the depolarization of these same neurons blunted the response to stimulation.

With the knowledge that the T11-T13 segments preferentially activate sympathetic structures and stimulation of these segments can modulate blood pressure, high resolution CT and MRI scans were performed to accurately identify the relationship between spinal segments and vertebral levels.

Additionally, the exact length of the T11-T13 segments using ex-vivo dissections has been confirmed.

Then, biocompatible electronic lead 20 spinal implants were designed with the exact dimensions required to stimulate T11-T13, with the lead 20 placed immediately under the T9-T12 vertebra, cf. Fig. 3a-3d.

This design of a lead 20 could thus be easily scaled to any animal or human model using MRI technology and computational modelling.

In other words, the design of the lead 20 is based off key anatomical features (using a rat model as an animal model)
- The identification of functional cardiovascular 'hotspots' - cf. Fig. 4a-4c (T11 - T13)
- The finding that these 'cardiovascular hotspots' are aligned with the segmental density of sympathetic pre-ganglionic neurons;
- Completing of CT and MRI scans of the same rat spinal cord in order to align the features of the lead 20 to the posterior roots of the T11-T13 segments (cf. Fig. 3a-3d for exact dimensions);
- Placing specific markers on the lead 20 to align the lead 20 to the specific vertebral locations;

Thus, the lead 20 dimensions (c.f. Fig. 3a-3d) are designed to perfectly target the posterior roots of the T11-T13 spinal segments of rats, which have determined are critical for the maintenance of blood pressure using epidural electrical stimulation.

In a human being, this would correspond to the region of T9-T12.

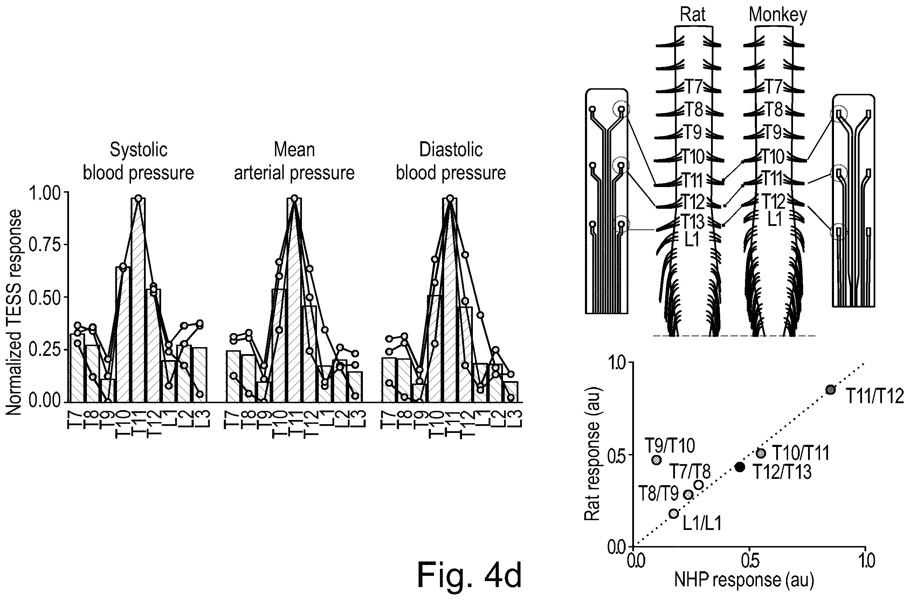

**Fig. 4d** shows the functional mapping in a non-human primate and demonstrates the conservation of the hotspot (please cf. also Fig. 3a-3d).

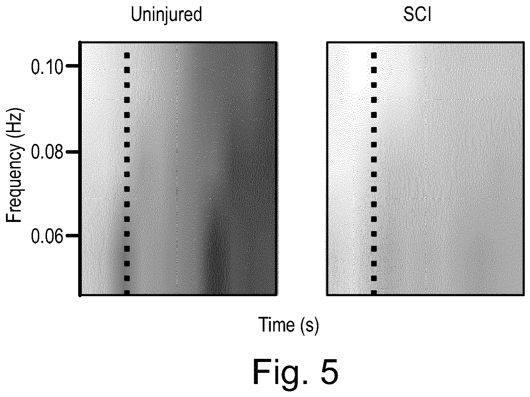

**Fig. 5** shows activation of natural frequency aspects within the systolic blood pressure signal during an orthostatic stimulus in uninjured and SCI rats.

Heat represents the frequency power at a given wavelet band.

The dotted line represents the onset of the orthostatic stimulus.

30s of data are shown.

A decrease in the power within the range around 0.1 Hz is observed.

A stimulation paradigm could be designed that mimics the natural state of the intact sympathetic nervous system.

Specifically, frequency oscillation overlays could be optimized, which in an intact system originate in supraspinal/spinal structures responsible for blood pressure control (i.e., the rostral ventrolateral medulla/spinal cord), and elicit a 0.1Hz low frequency oscillation sympathetic pre-ganglionic neurons.

Not shown is that, using electrophysiological experiments, it was determined that action potentials originating in the rostral ventrolateral medulla travel with a time delay of 2ms between T11-T12 and T12-T13 in a rat.

In a human being, this would correspond to the region of T9-T11 and T11-T12.

A biomimetic stimulation paradigm is described that reproduces the natural supraspinal sympathetic drive and when coupled with a range of standard parameters (frequency of 10Hz-10kHz, amplitude of 0-1A or 0-15V, pulse width of 1-500µs), achieves biologically relevant BIO control over blood pressure after spinal cord injury.

It was confirmed that this BIO paradigm recapitulates natural dynamics of the autonomic nervous system using wavelet decomposition, where an increase in the frequency power within the systolic blood pressure signal upon activation of the stimulation is observed.

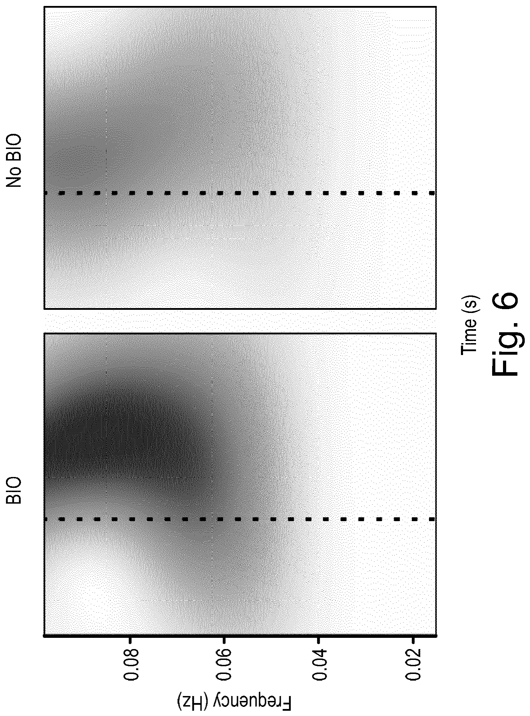

**Fig. 6** shows activation of natural frequency aspects within the systolic blood pressure signal using biologically relevant BIO stimulation.

Heat represents the frequency power at a given wavelet band.

The dotted line represents the onset of the stimulation.

30s of data are shown.

An increase in the power within the range around 0.1Hz was observed, confirming that the natural rhythm found in the uninjured state was recapitulated.

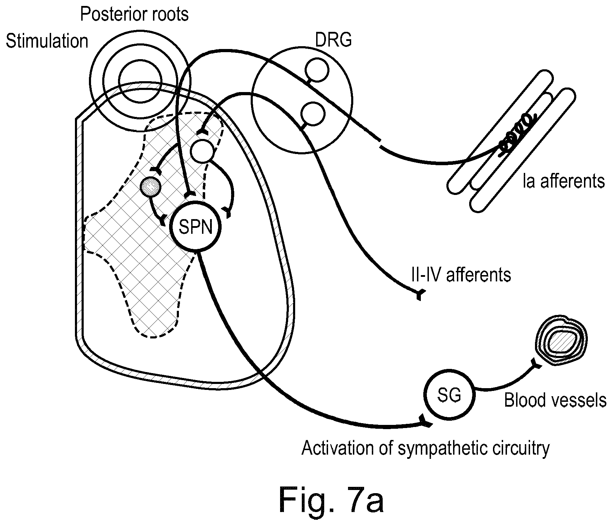

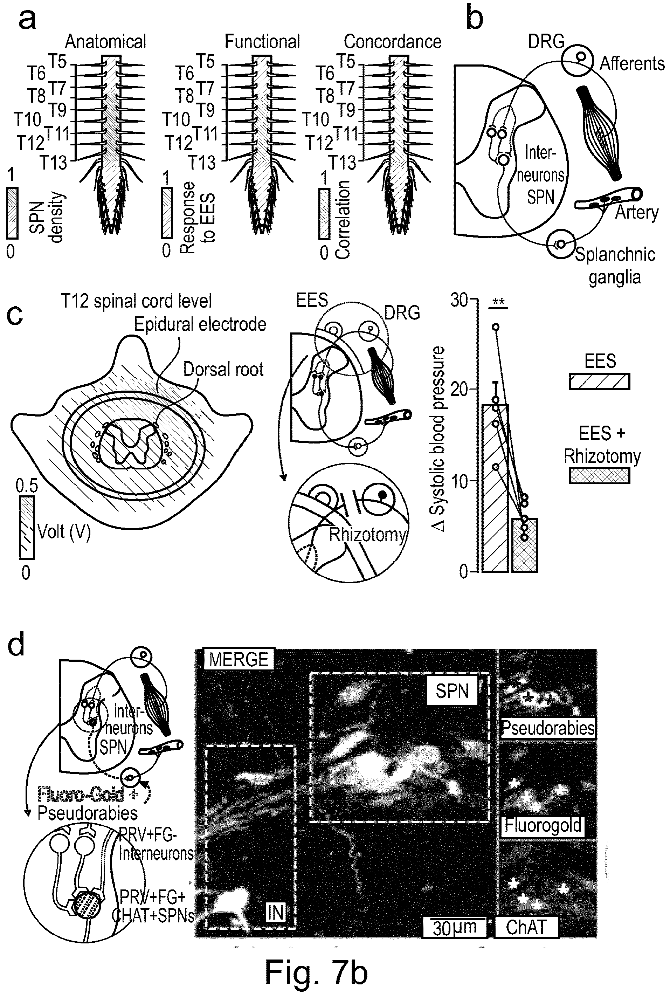

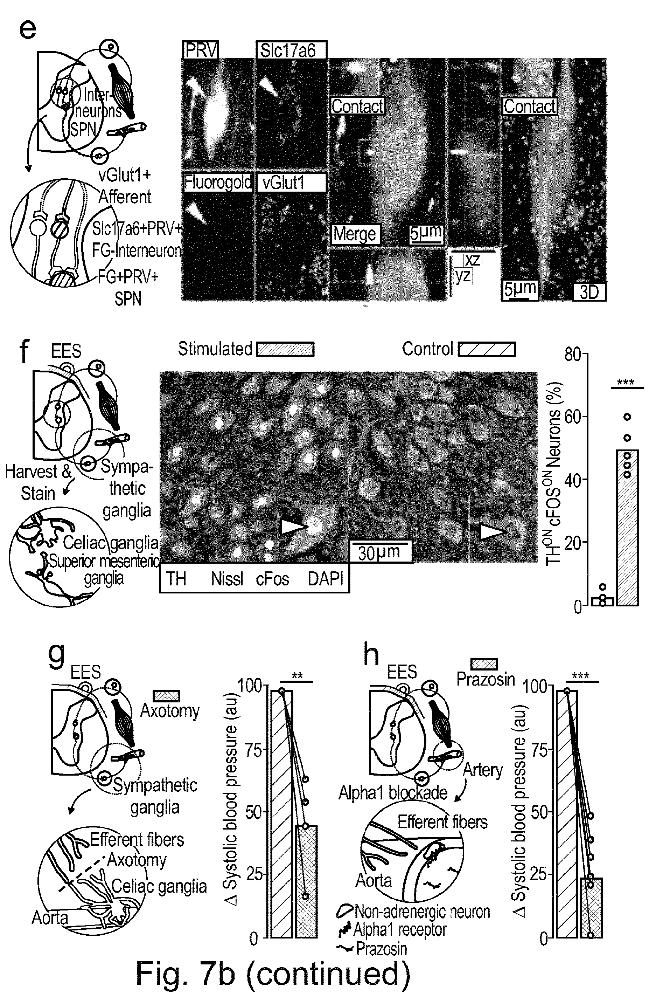

**Fig. 7a** shows a schematical overview of the activation of sympathetic neurons according to the present invention.

In particular, the activation of the sympathetic circuitry in response to stimulation is shown.

In particular, the activation of the sympathetic circuitry in response to stimulation with the system 10 and/or the method according to the present invention is shown.

Stimulation enters through the posterior roots of the dorsal root ganglion DRG and activates sympathetic pre-ganglionic neurons SPN, which then activate splanchic ganglia SG responsible for blood pressure.

**Fig. 7b** shows a schematical overview of mechanisms, by which EES stabilizes hemodynamics.

**Part a** shows the intraspinal density of neurons retrogradely traced from the splanchnic ganglia, amplitude of pressor responses to TESS applied to each segment, and concordance between anatomical and functional datasets.

**Part b** shows hypothetical circuits activated by TESS to elicit blood vessel constriction. **Part c** shows color-coded electrical potentials following TESS applied to the spinal cord suggesting the exclusive activation of afferent fibres. Scheme illustrating rhizotomy of posterior roots. Barplots report pressor responses to Targeted Epidural Spinal Stimulation (TESS) before and after rhizotomy (n = 5, paired samples one-tailed t-test; t = 4.36; P = 0.006). **Part d** shows trans-synaptic retrograde tracing revealing interneurons connected to splanchnic ganglia.

**Part e** shows interneurons. These interneurons express the excitatory marker Slc17a6, and receive vGlut1 synapses from large-diameter proprioceptive afferents. **Part f** shows Fos expression in THON neurons in the splanchnic ganglia in control and after TESS. Barplot reports percentage of FOSON neurons (n = 5, independent samples one-tailed t-test; t = 13.96; P = 4.99e-05). **Part g** shows ablation of splanchnic efferents blunted the pressor response (n = 4, independent samples one-tailed t-test; t = -4.54; P = 0.0099). **Part h** shows alpha1 receptor blockade with prazosin blunted pressor responses (n = 5, independent samples one-tailed t-test; t = -5.59; P = 0.0007).

### References

- 10: system
- 12: control unit
- 14: stimulation unit
- 16: signal processing unit
- 18: real-time monitoring unit
- 18a: sensor unit
- 20: lead

- BIO: biologically relevant stimulation
- CT: computer tomography
- DRG: dorsal root ganglion
- MRI: magnetic resonance imaging
- P: patient
- SCI: spinal cord injury
- SPN: sympathetic pre-ganglionic neurons
- SG: splanchic ganglia

- WL: wireless link

- DBP: diastolic blood pressure
- MAP: mean arterial pressure
- SBP: systolic blood pressure

- LX: lumbar vertebra level X
- L1: lumbar vertebra level 1
- TX: thoracic spinal segmental level X or thoracic vertebra level X
- T6: thoracic spinal segmental level 6 or thoracic vertebra level 6
- T7: thoracic spinal segmental level 7 or thoracic vertebra level 7
- T8: thoracic spinal segmental level 8 or thoracic vertebra level 8
- T9: thoracic spinal segmental level 9 or thoracic vertebra level 9
- T10: thoracic spinal segmental level 10 or thoracic vertebra level 10
- T11: thoracic spinal segmental level 11 or thoracic vertebra level 11
- T12: thoracic spinal segmental level 12 or thoracic vertebra level 12
- T13: thoracic spinal segmental level 13 or thoracic vertebra level 12

## Claims

1. A system (10) for activating a sympathetic circuitry in response to electrical stimulation, comprising:
at least one stimulation unit (14) comprising a lead (20) for electrical stimulation of a spinal cord,
**characterized in that**
the lead (20) is configured to stimulate functional cardiovascular hotspots in the spinal cord, and
wherein the functional cardiovascular hotspots are optimal locations on the spinal cord for eliciting blood pressure responses, wherein the optimal locations are preidentified through functional mapping of the spinal cord by stimulating the spinal segments from T5 to L2, and
wherein the lead (20) comprises electrodes that are designed to specifically stimulate said hotspots to target sympathetic pre- and post-ganglionic neurons responsible for blood pressure control.

2. The system (10) according to claim 1,
**characterized in that**
the lead (20) is capable and configured to provide stimulation to the spinal cord at level of vertebrate T9-T12, wherein the levels T9-T12 are aligned with the functional cardiovascular hotspots in the spinal cord.

3. The system (10) according to one of the preceding claims,
**characterized in that**
the lead (20) is capable and configured to be positioned subdurally and/or epidurally at least partially at and/or between the level of vertebrae T9-L1, in particular at least partially under vertebrae T9-L1.

4. The system (10) according to one of the preceding claims,
**characterized in that**
the system further comprises at least one control unit (12) that is configured and arranged to provide stimulation data.

5. The system (10) according to one of the preceding claims,
**characterized in that**
the at least one control unit (12) is further configured to provide a biomimetic stimulation paradigm that reproduces a natural supraspinal sympathetic drive.

6. The system (10) according to one of claims 4 or 5,
**characterized in that**
the at least one control unit (12) further comprises an oscillation control module, wherein the oscillation control module is configured and arranged to provide an input of 0.01Hz-0.2Hz low frequency oscillation in the amplitude and/or frequency.

7. The system (10) according to claim 6,
**characterized in that**
the oscillation control module is configured and arranged to provide an input of 0.1 Hz low frequency oscillation in the amplitude and/or frequency.

8. The system (10) according to one of claims 4 to 6,
**characterized in that**
the at least one control unit (12) further comprises a time control module,
wherein the time control module is configured and arranged to provide a time delay

9. The system (10) according to claim 8,
**characterized in that**
the time delay provided by the time control module is a time delay of 1-50 ms.

10. The system (10) according to one of claims 4 to 8,
**characterized in that**
the stimulation data comprise at least frequency, amplitude and pulse width, and wherein the frequency is 10Hz-10kHz, the amplitude is 0-1A or 0-15V and the pulse width is 1-500 µs.

11. The system (10) according to one of the preceding claims,
**characterized in that**
the stimulation unit (14) is configured and arranged to provide at least one burst train stimulation pulse.

12. The system (10) according to claim 11.
**characterized in that**
the stimulation unit (14) is configured and arranged to provide at least one burst of several pulses, preferably of 2 to 5 pulses.

13. The system (10) according to one of the preceding claims,
**characterized in that**
the functional cardiovascular hotspots correspond to the T11-T13 spinal segments.

14. The system (10) according to one of the preceding claims,
**characterized in that**
the lead (20) comprises markers on the electrodes to align the electrodes to specific vertebral locations.

## Patentansprüche

1. System (10) zum Aktivieren einer sympathischen Nervenbahn als Reaktion auf elektrische Stimulation, umfassend:
mindestens eine Stimulationseinheit (14) umfassend eine Leitung (20) zur elektrischen Stimulation eines Rückenmarks,
**dadurch gekennzeichnet, dass**
die Leitung (20) konfiguriert ist, um funktionelle kardiovaskuläre Hotspots im Rückenmark zu stimulieren, und
wobei die funktionellen kardiovaskulären Hotspots optimale Stellen im Rückenmark sind, um Blutdruckreaktionen hervorzurufen, wobei die optimalen Stellen durch funktionelle Kartierung des Rückenmarks durch Stimulation der Rückenmarkssegmente von T5 bis L2 vorab identifiziert werden, und
wobei die Leitung (20) Elektroden umfasst, die dazu bestimmt sind, die Hotspots gezielt zu stimulieren, um sympathische prä- und postganglionäre Neuronen anzusprechen, die für die Blutdruckkontrolle verantwortlich sind.

2. System (10) nach Anspruch 1,
**dadurch gekennzeichnet, dass**
die Leitung (20) in der Lage und konfiguriert ist, um eine Stimulation des Rückenmarks auf Höhe der Wirbel T9-T12 bereitzustellen, wobei die Höhen T9-T12 an den funktionellen kardiovaskulären Hotspots im Rückenmark ausgerichtet sind.

3. System (10) nach einem der vorstehenden Ansprüche,
**dadurch gekennzeichnet, dass**
die Leitung (20) in der Lage und konfiguriert ist, um subdural und/oder epidural mindestens teilweise auf und/oder zwischen der Höhe der Wirbel T9-L1, insbesondere mindestens teilweise unter den Wirbeln T9-L1, positioniert zu werden.

4. System (10) nach einem der vorstehenden Ansprüche,
**dadurch gekennzeichnet, dass**
das System ferner mindestens eine Steuereinheit (12) umfasst, die konfiguriert und angeordnet ist, um Stimulationsdaten bereitzustellen.

5. System (10) nach einem der vorstehenden Ansprüche,
**dadurch gekennzeichnet, dass**
die mindestens eine Steuereinheit (12) ferner konfiguriert ist, um ein biomimetisches Stimulationsparadigma bereitzustellen, das eine natürliche supraspinale sympathische Ansteuerung reproduziert.

6. System (10) nach einem der Ansprüche 4 oder 5,
**dadurch gekennzeichnet, dass**
die mindestens eine Steuereinheit (12) ferner ein Schwingungssteuermodul umfasst, wobei das Schwingungssteuermodul konfiguriert und angeordnet ist, um eine Eingabe einer Niederfrequenzschwingung von 0,01 Hz-0,2 Hz in der Amplitude und/oder Frequenz bereitzustellen.

7. System (10) nach Anspruch 6,
**dadurch gekennzeichnet, dass**
das Schwingungssteuermodul konfiguriert und angeordnet ist, um eine Eingabe einer Niederfrequenzschwingung von 0,1 Hz in der Amplitude und/oder Frequenz bereitzustellen.

8. System (10) nach einem der Ansprüche 4 bis 6,
**dadurch gekennzeichnet, dass**
die mindestens eine Steuereinheit (12) ferner ein Zeitsteuermodul umfasst,
wobei das Zeitsteuermodul konfiguriert und angeordnet ist, um eine Zeitverzögerung bereitzustellen

9. System (10) nach Anspruch 8,
**dadurch gekennzeichnet, dass**
die vom Zeitsteuermodul bereitgestellte Zeitverzögerung 1-50 ms beträgt.

10. System (10) nach einem der Ansprüche 4 bis 8,
**dadurch gekennzeichnet, dass**
die Stimulationsdaten mindestens Frequenz, Amplitude und Impulsbreite umfassen und wobei die Frequenz 10 Hz-10 kHz, die Amplitude 0-1 A oder 0-15 V und die Impulsbreite 1-500 µs beträgt.

11. System (10) nach einem der vorstehenden Ansprüche,
**dadurch gekennzeichnet, dass**
die Stimulationseinheit (14) konfiguriert und angeordnet ist, um mindestens einen Burst-Train-Stimulationsimpuls bereitzustellen.

12. System (10) nach Anspruch 11,
**dadurch gekennzeichnet, dass**
die Stimulationseinheit (14) konfiguriert und angeordnet ist, um mindestens einen Burst aus mehreren Impulsen, vorzugsweise aus 2 bis 5 Impulsen, bereitzustellen.

13. System (10) nach einem der vorstehenden Ansprüche,
**dadurch gekennzeichnet, dass**
die funktionellen kardiovaskulären Hotspots den Wirbelsäulensegmenten T11-T13 entsprechen.

14. System (10) nach einem der vorstehenden Ansprüche,
**dadurch gekennzeichnet, dass**
die Leitung (20) Markierungen auf den Elektroden umfasst, um die Elektroden an bestimmten Wirbelpositionen auszurichten.

## Revendications

1. Système (10) permettant d'activer une circuiterie sympathique en réponse à une stimulation électrique :
au moins une unité de stimulation (14) comprenant une sonde (20) pour une stimulation électrique d'une moelle épinière,
**caractérisé en ce que**
la sonde (20) est configurée pour stimuler des zones sensibles cardiovasculaires fonctionnelles dans la moelle épinière, et
dans lequel les zones sensibles cardiovasculaires fonctionnelles sont des emplacements optimaux de la moelle épinière pour susciter des réactions au niveau pression artérielle, dans lequel les emplacements optimaux sont identifiés au préalable par cartographie fonctionnelle de la moelle épinière en stimulant les segments spinaux T5 à L2, et
dans lequel la sonde (20) comprend des électrodes configurées pour stimuler spécifiquement lesdites zones sensibles afin de cibler des neurones pré et postganglionnaires sympathiques responsables de la régulation de pression artérielle.

2. Système (10) selon la revendication 1,
**caractérisé en ce que**
la sonde (20) est adaptée et configurée pour fournir une stimulation à la moelle épinière au niveau des vertèbres T9 à T12, dans lequel les niveaux T9 à T12 sont alignés avec les zones sensibles cardiovasculaires fonctionnelles de la moelle épinière.

3. Système (10) selon l'une des revendications précédentes,
**caractérisé en ce que**
la sonde (20) est adaptée et conçue pour être positionnée par voie sous-durale et/ou épidurale au moins partiellement au niveau des vertèbres T9 à L1 et/ou entre celles-ci, en particulier au moins partiellement sous les vertèbres T9 à L1.

4. Système (10) selon l'une des revendications précédentes,
**caractérisé en ce que**
le système comprend en outre au moins une unité de commande (12) configurée et agencée pour fournir des données de stimulation.

5. Système (10) selon l'une des revendications précédentes,
**caractérisé en ce que**
l'au moins une unité de commande (12) est en outre configurée pour fournir un paradigme de stimulation biomimétique qui reproduit une conduction sympathique supraspinale naturelle.

6. Système (10) selon l'une des revendications 4 ou 5,
**caractérisé en ce que**
l'au moins une unité de commande (12) comprend en outre un module de commande d'oscillation, dans lequel le module de commande d'oscillation est configuré et agencé pour fournir une entrée d'oscillation basse fréquence de 0,01 Hz à 0,2 Hz dans l'amplitude et/ou la fréquence.

7. Système (10) selon la revendication 6,
**caractérisé en ce que**
le module de commande d'oscillation est configuré et agencé pour fournir une entrée d'oscillation basse fréquence de 0,1 Hz dans l'amplitude et/ou la fréquence.

8. Système (10) selon l'une des revendications 4 à 6,
**caractérisé en ce que**
l'au moins une unité de commande (12) comprend en outre un module de commande temporelle,
dans lequel le module de commande temporelle est configuré et agencé pour fournir une temporisation

9. Système (10) selon la revendication 8,
**caractérisé en ce que**
la temporisation fournie par le module de commande temporelle est une temporisation de 1 à 50 ms.

10. Système (10) selon l'une des revendications 4 à 8,
**caractérisé en ce que**
les données de stimulation comprennent au moins une fréquence, amplitude et durée d'impulsion, et dans lequel la fréquence est de 10 Hz à 10 kHz, l'amplitude est de 0 à 1 A ou de 0 à 15 V et la durée d'impulsion est de 1 à 500 µs.

11. Système (10) selon l'une des revendications précédentes,
**caractérisé en ce que**
l'unité de stimulation (14) est configurée et agencée pour fournir au moins une impulsion de stimulation par train de rafales.

12. Système (10) selon la revendication 11,
**caractérisé en ce que**
l'unité de stimulation (14) est configurée et agencée pour fournir au moins une rafale de plusieurs impulsions, de préférence de 2 à 5 impulsions.

13. Système (10) selon l'une des revendications précédentes,
**caractérisé en ce que**
les zones sensibles cardiovasculaires fonctionnelles correspondent aux segments spinaux T11 à T13.

14. Système (10) selon l'une des revendications précédentes,
**caractérisé en ce que**
la sonde (20) comprend des marqueurs sur les électrodes afin d'aligner les électrodes avec des emplacements vertébraux spécifiques.
